(19) **Europäisches Patentamt**

**European Patent Office**

**Office européen des brevets**

(11) Veröffentlichungsnummer: **0 328 758 B1**

(12) # EUROPÄISCHE PATENTSCHRIFT

(45) Veröffentlichungstag der Patentschrift: **15.04.92**

(51) Int. Cl.⁵: **B01D 53/34**

(21) Anmeldenummer: **88120404.4**

(22) Anmeldetag: **07.12.88**

(54) **Verfahren und Vorrichtung zur biologischen Reinigung von Abgas.**

(30) Priorität: **12.12.87 DE 3742219**

(43) Veröffentlichungstag der Anmeldung:
**23.08.89 Patentblatt 89/34**

(45) Bekanntmachung des Hinweises auf die
Patenterteilung:
**15.04.92 Patentblatt 92/16**

(84) Benannte Vertragsstaaten:
**AT BE CH DE FR IT LI NL SE**

(56) Entgegenhaltungen:
**EP-A- 0 100 024**
**DE-A- 2 839 173**
**DE-A- 3 423 285**

(73) Patentinhaber: **Linde Aktiengesellschaft**
**Abraham-Lincoln-Strasse 21**
**W-6200 Wiesbaden(DE)**

(72) Erfinder: **Fuchs, Uwe, Chem.-Ing.**
**Heiterwanger Strasse 46**
**W-8000 München 70(DE)**

(74) Vertreter: **Schaefer, Gerhard, Dr.**
**Linde Aktiengesellschaft Zentrale Patentab-**
**teilung**
**W-8023 Höllriegelskreuth(DE)**

EP 0 328 758 B1

Rank Xerox (UK) Business Services

## Beschreibung

Die Erfindung betrifft ein Verfahren zur Reinigung von Abgas, bei dem unerwünschte Abgasbestandteile durch Mikroorganismen in unschädliche Produkte umgewandelt werden.

Aus der DE-A-34 23 285 ist bereits ein Verfahren zur biologischen Reinigung von Abgas bekannt. Bei diesem Verfahren wird stickoxidhaltiges Abgas dadurch gereinigt, daß die Stickoxide unter anoxischen Bedingungen in Gegenwart eines oxidierbare organische Kohlenstoffverbindungen enthaltenden Subtrats von in einem Reaktor auf speziellen Trägerelementen fixierten Mikroorganismen zu unschädlichem Stickstoff reduziert werden. Dabei sind die Trägerelemente in dem Reaktor fest installiert und werden von einer organische Kohlenstoffverbindungen aufweisenden Flüssigkeit (z.B. kommunales Abwasser) berieselt. Das zu reinigende Abgas wird durch den Reaktor hindurchgeleitet.

Dieses bekannte Verfahren ist also speziell auf die Elimination von Stickoxiden aus Abgas abgestimmt.

Insbesondere Abgase aus Industriebetrieben, wie z.B. Gießereien, Glasschmelzen, Mineralfaserproduktionsstätten, Lackierereien usw., weisen jedoch in zunehmendem Maße hohe Konzentrationen von schwer abbaubaren Problemstoffen auf.

Aufgabe der vorliegenden Erfindung ist es daher, ein Verfahren und eine Vorrichtung der eingangs genannten Art so auszugestalten, daß auf einfache und wirtschaftliche Weise eine hohe Reinigungsleistung, insbesondere bei der Reinigung Problemstoffe enthaltenden Abgases, erreicht wird.

Diese Aufgabe wird erfindungsgemäß dadurch gelöst, daß die unerwünschten Abgasbestandteile mindestens einem flüssigkeitsgefüllten Reaktor zugeführt werden, wobei einzelne Tragerteilchen in stückiger und/oder granulierter Form, auf denen Mikroorganismen angesiedelt sind, frei beweglich in der Flüssigkeit schweben.

Die unerwünschten Abgasbestandteile lösen sich in der Flüssigkeit und treten mit den in der Flüssigkeit auf den frei schwebenden Trägerteilchen angesiedelten Mikroorganismen in Wechselwirkung. Da die Mikroorganismen von allen Seiten von den Abgasbestandteilen und der Flüssigkeit gut zugänglich sind, ist ein hoher Stoffaustausch und damit eine hohe Reinigungsleistung gewährleistet.

Um die Schwebebewegung der einzelnen Trägerteilchen nicht zu behindern und eine Verwirbelung der Trägerteilchen zu ermöglichen, ist es zweckmäßig, die Trägerteilchen in einer Menge in den Reaktor einzubringen, die einem Volumenanteil von 5 bis 40% des Reaktorvolumens entspricht. Die Verwirbelung kann durch ein Rühraggregat verstärkt werden, das Turbulenzen in der Flüssigkeit erzeugt.

Die Reinigungsleistung kann in einer besonders bevorzugten Ausgestaltung der Erfindung noch dadurch gesteigert werden, daß als Trägerteilchen Stoffteilchen mit einer Größe von 5 bis 55 mm, mit offenen Makroporen von 0,1 bis 3 mm und mit einem spezifischen Gewicht im trockenen Zustand von 10 bis 200 $kg/m^3$ verwendet werden.

Aufgrund der offenen Makroporen werden die Mikroorganismen in hoher Konzentration angereichert und zu einem dezentralisierten Wachstum gezwungen, so daß in Verbindung mit der Größe der Stoffteilchen eine besonders gute Zugänglichkeit für die Abgasbestandteile und die Flüssigkeit zu den Mikroorganismen gegeben ist. Die angegebene Größe der Stoffteilchen führt zu stabilen Bakterienansammlungen und gewährleistet einen Stofftransport bis ins Innere jedes einzelnen Stoffteilchens. Die Größe der einzelnen Makroporen in den Stoffteilchen schließt ein Verstopfen der Poren durch Verunreinigungen weitgehend aus und ermöglicht es, den Mikroorganismen eine große Oberfläche zur Ansiedlung zur Verfügung zu stellen. Das spezifische Gewicht von 10 bis 200 $kg/m^3$ bewirkt außerdem eine gleichmäßige Verteilung der Trägerteilchen in der Flüssigkeit. Insgesamt wird also ein intensiver Stoffaustausch zwischen Mikroorganismen, Abgasbestandteilen und der Flüssigkeit erreicht.

Zweckmäßigerweise wird für die Füllung des Reaktors eine wäßrige, Nährsalze für die Mikroorganismen enthaltende Lösung verwendet. Die für eine intensive Mikroorganismentätigkeit und damit hohen Stoffumsatz erforderlichen Nährstoffe können so direkt den Mikroorganismen zugeführt werden.

Für die meisten Anwendungsfälle ist eine aerobe Betriebsweise des Reaktors zur Elimination der unerwünschten Abgasbestandteile vorgesehen. Dabei werden die Abgasbestandteile durch die Mikroorganismentätigkeit zu unschädlichen Produkten oxidiert und/oder in die Körpersubstanz der Mikroorganismen aufgenommen.

Der für die aerobe Betriebsweise nötige Sauerstoff kann bereits im Abgas enthalten sein oder er wird in Form von Luft oder mit Sauerstoff angereichertem Gas in die im Reaktor befindliche Flüssigkeit eingetragen. Grundsätzlich ist es dabei vorteilhaft, den Sauerstoffeintrag so zu gestalten, daß in der Flüssigkeit Turbulenzen auftreten. Durch eine Verwirbelung der Trägerteilchen in der Flüssigkeit wird nämlich der Stoffaustausch noch wesentlich erhöht.

Das Verfahren eignet sich insbesondere für die Reinigung ammoniakhaltigen und/oder Phenol-Formaldehyd-Verbindungen enthaltenden Abgases. Die für den Abbau derartiger Problemstoffe geeigneten Mikroorganismen sind besonders sessiler Natur und benötigen unbedingt eine feste Ansiedlungsfläche. Andererseits sind sie darauf angewiesen, daß ihnen alle Nährstoffe und die abzubauenden Problemstoffe sowie der nötige Sauerstoff in gelöster Form zur Verfügung gestellt werden. Beide Voraussetzungen sind aber beim erfindungsgemäßen Verfahren erfüllt. Durch die freie Beweglichkeit der Trägerteilchen in der Flüssigkeit ist dabei ein besonders intensiver Stoffaustausch zwischen Mikroorganismen, Nährstoffen und Abgasbestandteilen gewährleistet.

Da die unerwünschten Abgasbestandteile erfindungsgemäß in die im Reaktor befindliche Flüssigkeit eingeleitet werden, ist die Verweilzeit der Abgasbestandteile im Reaktor gegenüber abgasdurchströmten Festbettreaktoren erhöht. Andererseits werden die Mikroorganismen durch deren Ansiedlung auf den Trägerteilchen genügend lange im Reaktor gehalten, um eine weitgehende Eliminierung der unerwünschten Abgasbestandteile durch Mikroorganismen zu gewährleisten. Da auf den Abbau von Problemstoffen spezialisierte Mikroorganismen für die Elimination dieser Stoffe längere Zeit benötigen als z.B. auf den Abbau organischer Verunreinigungen ausgerichtete Bakterien zum Abbau der organischen Substanzen, wirkt sich die mit dem erfindungsgemäßen Verfahren erreichbare lange Aufenthaltszeit von Abgasbestandteilen und Mikroorganismen im Reaktor besonders bei der Behandlung Problemstoffe enthaltenden Abgases positiv auf die Reinigungsleistung aus. Darüber hinaus kann durch eine zusätzliche Kreislaufführung die Aufenthaltszeit der Abgasbestandteile noch verlängert werden.

In einer bevorzugten Ausführungsform der Erfindung werden die unerwünschten Abgasbestandteile in einem dem Reaktor vorgeschalteten Wäscher zunächst durch Besprühen mit der Flüssigkeit aus dem Abgas ausgewaschen. Zweckmäßigerweise wird hierfür ein Teil der Flüssigkeit aus dem Reaktor abgezogen und über Sprühdüsen im Wäscher versprüht. In den Wäscher wird das zu reinigende Abgas eingeleitet und gereinigtes Abgas abgezogen. Die aus dem Abgas ausgewaschenen Abgasbestandteile werden zusammen mit der Flüssigkeit dem Reaktor zugeführt. Dort erfolgt dann der eigentlich Abbau der unerwünschten Abgasbestandteile durch Mikroorganismen.

Falls Stickstoffverbindungen enthaltendes, z.B. stark ammoniakhaltiges, Abgas gereinigt werden soll, so kann gemäß einer weiteren Ausgestaltung des erfindungsgemäßen Verfahrens dem unter aeroben Bedingungen arbeitenden Reaktor eine unter anoxischen Bedingungen arbeitende Denitrifikationsstufe vorgeschaltet werden. Die vom Wäscher abgezogene mit den unerwünschten Abgasbestandteilen beladene Flüssigkeit wird zunächst in die Denitrifikationsstufe eingeleitet und anschließend in den Reaktor. Da im Reaktor je nach Belastung neben den auf Trägerteilchen fixierten Mikroorganismen auch freie Mikroorganismen enthaltender Belebtschlamm entsteht, kann ein Teil des Belebtschlamms in die Denitrifikationsstufe zur Animpfung zurückgeführt werden. Außerdem wird ein Teil der aus dem Reaktor abgezogenen Flüssigkeit in die Denitrifikationsstufe zurückgeleitet. Im Reaktor werden die Stickstoffverbindungen zu Stickoxiden oxidiert, die mit der rückgeführten Flüssigkeit in die Denitrifikationsstufe gelangen. Dort werden sie zu elementarem Stickstoff reduziert, der bedenkenlos an die Atmosphäre abgegeben werden kann.

In Weiterführung des Erfindungsgedankens ist ferner vorgesehen, einen Teil des im Reaktor entstandenen Belebtschlamms zusammen mit der Flüssigkeit im Wäscher zu versprühen. Dadurch ist es möglich, auch den Wäscher biologisch zu betreiben und bereits dort erste die Umwandlungsschritte einzuleiten.

Bei einem aeroben Betrieb des Reaktors ist es besonders vorteilhaft, die Luft oder das mit Sauerstoff angereicherte Gas in der Weise in den Reaktor einzuleiten, daß in der Flüssigkeit Turbulenzen entstehen. Dadurch wird der Stoffaustausch zwischen Mikroorganismen, Abgasbestandteilen und Flüssigkeit wesentlich erhöht. Zweckmäßigerweise erfolgt dabei der Eintrag des Gases über am Boden des Reaktors angebrachte Düsen.

Eine andere Ausgestaltung der Erfindung besteht darin, daß das Abgas nicht zuerst einem Wäscher, sondern direkt dem Reaktor zugeführt wird. Das gesamte Abgas wird dabei unmittelbar in die im Reaktor befindliche Flüssigkeit eingetragen. Die unerwünschten Abgasbestandteile lösen sich zum Teil in der Flüssigkeit und werden von den Mikroorganismen abgebaut. Auch hier ist wieder die Erzeugung von Turbulenzen in der Flüssigkeit erwünscht, um den Stoffaustausch zu intensivieren. Deshalb wird zweckmäßigerweise das Abgas am Boden des Reaktors über Düsen in die Flüssigkeit so eingetragen, daß eine Verwirbelung der in der Flüssigkeit schwebenden Trägerteilchen erfolgt, die mit einem Zusatzrühraggregat noch verstärkt werden kann.

Falls die zu entfernenden Abgasbestandteile eine aerobe Behandlung erfordern und der nötige Sauerstoff nicht von vornherein im Abgas enthalten ist, so wird vorteilhafterweise Luft oder ein mit Sauerstoff angereichertes Gas dem Abgas beigemengt, bevor es in den Reaktor eingeleitet wird.

Eine Vorrichtung zur Durchführung des Verfahrens besteht aus mindestens einem Reaktor mit einer Zuleitung für die zu entfernenden unerwünschten Abgasbestandteile.

Erfindungsgemäß ist der Reaktor mit einer Flüssigkeit gefüllt, in der einzelne makroporöse Stoffteilchen, auf denen in Mikroorganismen angesiedelt sind, in stückiger und/oder granulierter Form frei beweglich schweben.

In einer zweckmäßigen Ausführungsform ist dem Reaktor ein Sprühturm vorgeschaltet, der eine Zuleitung für zu reinigendes Abgas und eine Ableitung für gereinigtes Abgas aufweist. Der Sprühturm ist über die Zuleitung für die zu entfernenden unerwünschten Abgasbestandteile mit dem Reaktor verbunden. Außerdem weist der Sprühturm Sprühdüsen auf, die mit einer Zuleitung für die Flüssigkeit verbunden sind. Diese Zuleitung steht ihrerseits mit dem Reaktor in Verbindung, so daß Flüssigkeit aus dem Reaktor entnommen und im Sprühturm zum Auswaschen der unerwünschten Abgasbestandteile aus dem Abgas versprüht werden kann.

Die Erfindung eignet sich sowohl zur Reinigung organisch verunreinigten Abgases als auch schwer abbaubare Problemstoffe aufweisenden Abgases. Aber insbesondere bei der Reinigung von Abgasen mit speziellen Abgasinhaltsstoffen, die eine längere Zeit für einen biologischen Abbau durch darauf spezialisierte Mikroorganismen erfordern, kommen die Vorteile des erfindungsgemäßen Verfahrens und der erfindungsgemäßen Vorrichtung besonders zum Tragen. Solche Abgasinhaltsstoffe sind z.B. Ammoniak, Formaldehyd, Phenol, Amine usw. Somit eignet sich die Erfindung besonders für die Abgasreinigung bei Gießereien, Lackierereien, Glasschmelzen, Mineralfaserproduktionen etc.

Im folgenden soll die Erfindung anhand von in Zeichnungen schematisch dargestellten Ausführungsbeispielen näher erläutert werden:
Es zeigen:

Figur 1     ein Fließschema des erfindungsgemäßen Verfahrens bei direkter Einleitung des gesamten Abgases in den Reaktor.

Figur 2     ein Fließschema des erfindungsgemäßen Verfahrens mit vorgeschaltetem Wäscher.

Figur 3     ein Fließschema des erfindungsgemäßen Verfahrens mit vorgeschaltetem Wäscher und zusätzlicher Denitrifikationsstufe.

Figur 4     ein Fließschema des erfindungsgemäßen Verfahrens mit in den Reaktor integriertem Wäscher.

In den verschiedenen Figuren sind analoge Anlagenteile mit denselben Bezugszeichen versehen.

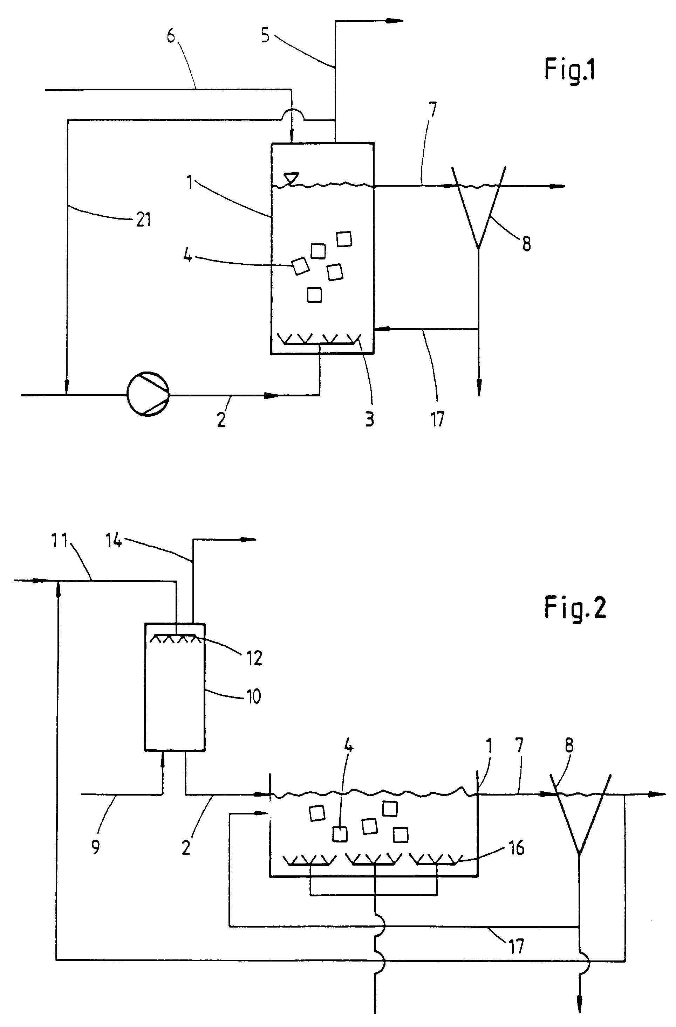

Bei dem in Figur 1 dargestellten Ausführungsbeispiel wird das gesamte zu reinigende Abgas direkt über Leitung 2 dem flüssigkeitsgefüllten Reaktor 1 zugeführt. Das Abgas wird über am Boden des Reaktors 1 angebrachte Düsen 3 unter erhöhtem Druck in die Flüssigkeit eingetragen. In der Flüssigkeit entstehen somit Turbulenzen, so daß eine Verwirbelung der in der Flüssigkeit schwebenden Trägerteilchen 4 erfolgt.

Die auf den Trägerteilchen 4 angesiedelten Mikroorganismen wandeln die unerwünschten Abgasbestandteile in unschädliche Produkte um, so daß gereinigtes Abgas über Leitung 5 abgezogen werden kann. Zumindest ein Teil des Abgases wird über Leitung 21 zur erneuten Reinigung wieder zum Reaktor 1 zurückgeführt.

Über Leitung 6 wird frische, Nährstoffe für die Mikroorganismen enthaltende Flüssigkeit zugeführt, während verbrauchte Flüssigkeit über Leitung 7 abgezogen wird. Als Nährstoffe kommen z.B. Phosphat, Spurenelemente, organische Verbindungen usw. in Frage. Da im Reaktor 1 neben den auf Trägerteilchen 4 fixierten Mikroorganismen freie Mikroorganismen enthaltender Belebtschlamm entsteht, ist eine Abtrennung des Belebtschlamms in einer dem Reaktor 1 nachgeschalteten Sedimentationsstufe 8 vorgesehen. Ein Teil des in der Sedimentationsstufe 8 abgesetzten Belebtschlamms wird über Leitung 17 in den Reaktor 1 zurückgeführt, der Rest als Überschußschlamm abgezogen.

Als Trägerteilchen 4 kommen Polyurethan-Schaumstoffwürfel mit offenen Makroporen von ca. 0,5 mm und einem spezifischen Gewicht im trockenen Zustand von 60 kg/m$^3$ zur Anwendung.

Die Schaumstoffwürfel weisen ei ne Kantenlänge von ca. 10 mm auf.

Figur 2 zeigt ein Fließschema einer anderen Variante des erfindungsgemäßen Verfahrens. Das zu reinigende Abgas wird zunächst über Leitung 9 einem Wäscher 10 zugeführt, in dem die unerwünschten Abgasbestandteile aus dem Abgas ausgewaschen werden. Hierzu wird Flüssigkeit, die aus dem Reaktor 1 entnommen wird, mit frischer, Nährstoffe für die Mikroorganismen enthaltender Flüssigkeit vermischt und über Leitung 11 den im Wäscher 10 installierten Sprühdüsen 12 zugeleitet. Gereinigtes Abgas wird über Leitung 14 abgezogen.

Die aus dem Abgas ausgewaschenen Abgasbestandteile werden zusammen mit der Flüssigkeit über Leitung 2 dem Reaktor 1 zugeführt. Dort erfolgt der eigentliche Abbau der unerwünschten Abgasbestandteile durch die auf den Trägerteilchen 4 fixierten Mikroorganismen. Durch Eintrag von Luft über die Düsen 16 wird eine Verwirbelung der in der Flüssigkeit schwebenden Trägerteilchen 4 und damit eine Intensivierung des Stoffaustauschs erreicht.

Verbrauchte Flüssigkeit wird über Leitung 7 aus dem Reaktor 1 abgezogen. In einer dem Reaktor 1 nachgeschalteten Sedimentationsstufe 8 wird analog zu dem in Zusammenhang mit Figur 1 beschriebenen Ausführungsbeispiel Belebtschlamm abgetrennt und teilweise in den Reaktor 1 zurückgeführt, teilweise als Überschußschlamm abgezogen.

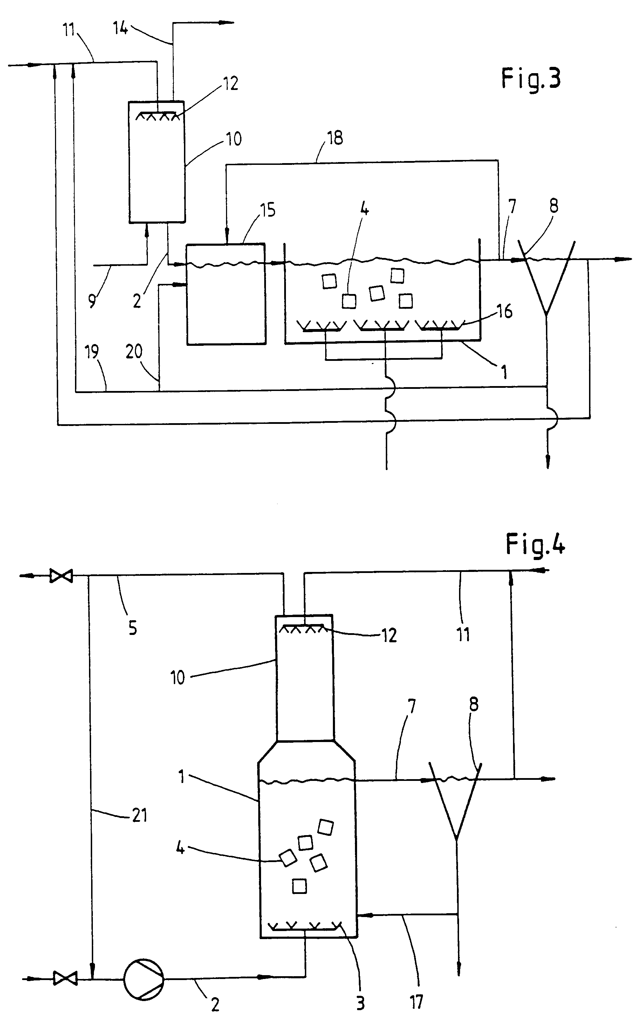

Figur 3 zeigt eine Weiterbildung des in Figur 2 gezeigten Ausführungsbeispiels. Zwischen dem Wäscher 10 und dem Reaktor ist zusätzlich eine unter anoxischen Bedingungen betriebene Denitrifikationsstufe 15 angeordnet, in der Nitrat und Nitrit zu elementarem Stickstoff unter Einwirkung von Mikroorganismen denitrifiziert werden. Hierzu wird ein Teil des aus der Sedimentationsstufe 8 abgetrennten Belebtschlamms über Leitung 20 zur Denitrifikationsstufe 15 zurückgeführt. Außerdem wird ein Teil der aus dem Reaktor 1 abgezogenen Flüssigkeit über Leitung 18 ebenfalls in die Denitrifikationsstufe 15 zurückgeleitet. Im Reaktor 1 durch mikrobielle Oxidation erzeugtes Nitrat und Nitrit gelangen mit der rückgeführten Flüssigkeit in die Denitrifikationsstufe 15, wo sie zu elementarem Stickstoff reduziert werden.

Eine weitere Steigerung der Reinigungsleistung wird dadurch erreicht, daß ein Teil des in der Sedimentationsstufe 8 abgesetzten Schlamms mit der dem Wäscher 10 zugeführten Flüssigkeit vermischt wird und gemeinsam mit dieser im Wäscher versprüht wird. Hierzu wird ein Teil des Schlamms über Leitung 19 zum Wäscher zurückgeleitet.

In Figur 4 ist eine weitere Variante des erfindungsgemäßen Verfahrens dargestellt. Dieses Ausführungsbeispiel entspricht einer Kombination der in den Figuren 1 und 2 gezeigten Verfahren. Das zu reinigende Abgas wird über Leitung 2 in die im Reaktor 1 befindliche Flüssigkeit unter erhöhtem Druck analog zu dem in Zusammenhang mit Figur 1 beschriebenen Ausführungsbeispiel eingetragen. Das aus dem Reaktor 1 entweichende Abgas wird nicht gleich, wie in Figur 1 gezeigt, über Leitung 5 abgezogen, sondern tritt zunächst in den über dem Reaktor 1 sich anschließenden Wäscher 10 über. Dort wird das Abgas durch Einsprühen von Flüssigkeit gewaschen, wobei die ausgewaschenen Abgasbestandteile wieder zurück in den Reaktor 1 gelangen und erneut einem biologischen Abbau unterzogen werden. Durch diese Maßnahme wird erreicht daß insbesondere leicht flüchtige Abgasbestandteile längere Zeit einem biologischen Abbau im Reaktor 1 zur Verfügung stehen.

Außerdem wird ein Teil des Abgases über Leitung 21 zum Reaktor 1 zurückgeführt, um durch eine Kreislaufführung des Abgases eine zusätzliche Erhöhung der Aufenthaltszeit der Abgasbestandteile im Reaktor 1 zu ermöglichen.

## Patentansprüche

1.   Verfahren zur Reinigung von Abgas, bei dem unerwünschte Abgasbestandteile durch Mikroorganismen in unschädliche Produkte umgewandelt werden, dadurch gekennzeichnet, daß die unerwünschten Abgasbestandteile mindestens einem flüssigkeitsgefüllten Reaktor (1) zugeführt werden, wobei einzelne Tragerteilchen (4) in stückiger und/oder granulierter Form, auf denen Mikrooganismen angesiedelt sind, frei beweglich in der Flüssigkeit schweben.

2.   Verfahren nach Anspruch 1, dadurch gekennzeichnet, daß in der Flüssigkeit Turbulenzen erzeugt werden.

3.   Verfahren nach Anspruch 1 oder 2, dadurch gekennzeichnet, daß als Trägerteilchen (4) Stoffteilchen mit einer Größe von 5 bis 50 mm, mit offenen Makroporen von 0,1 bis 3 mm und mit einem spezifischen Gewicht im trockenen Zustand von 10 bis 200 kg/m$^3$ verwendet werden.

4.   Verfahren nach einem der Ansprüche 1 bis 3, dadurch gekennzeichnet, daß das Abgas als unerwünschte Abgasbestandteile Ammoniak und/oder Phenol-Formaldehyd-Verbindungen enthält.

5.   Verfahren nach einem der Ansprüche 1 bis 4, dadurch gekennzeichnet, daß als Flüssigkeit eine wäßrige, Nährsalze für die Mikroorganismen enthaltende Lösung verwendet wird.

6.   Verfahren nach einem der Ansprüche 1 bis 5, dadurch gekennzeichnet, daß der Reaktor (1) unter aeroben Bedingungen betrieben wird.

7.   Verfahren nach einem der Ansprüche 1 bis 6, dadurch gekennzeichnet, daß die unerwünschten Abgasbestandteile in einem dem Reaktor (1) vorgeschalteten Wäscher (10) zunächst durch Besprühen mit Flüssigkeit aus dem Abgas ausgewaschen werden und zusammen mit der Flüssigkeit dem Reaktor (1) zugeführt werden.

8.   Verfahren nach Anspruch 6 oder 7, dadurch gekennzeichnet, daß dem unter aeroben Bedingungen betriebenen Reaktor (1) eine unter anoxischen Bedingungen arbeitende Dentrifikationsstufe (15) vorgeschaltet ist, in die freie Mikroorganismen enthaltender Belebtschlamm aus dem Reaktor (1) zurückgeführt wird.

9.   Verfahren nach Anspruch 7 oder 8, dadurch gekennzeichnet, daß freie Mikroorganismen enthaltender Belebtschlamm aus dem Reaktor (1) zusammen mit der Flüssigkeit im Wäscher (10) versprüht wird.

10.  Verfahren nach einem der Ansprüche 6 bis 9, dadurch gekennzeichnet, daß in den Reaktor (1) Luft oder ein mit Sauerstoff angereichertes Gas in der Weise eingeleitet wird, daß in der Flüssigkeit Turbulenzen entstehen.

11. Verfahren nach einem der Ansprüche 1 bis 5, dadurch gekennzeichnt, daß das gesamte Abgas direkt in die im Reaktor (1) befindliche Flüssigkeit eingetragen wird.

12. Verfahren nach Anspruch 11, dadurch gekennzeichnet, daß das Abgas in der Weise in die Flüssigkeit eingeleitet wird, daß Turbulenzen in der Flüssigkeit entstehen.

13. Verfahren nach Anspruch 11 oder 12, dadurch gekennzeichnet, daß dem Abgas ein sauerstoffhaltiges Gas beigemengt wird.

14. Verfahren nach einem der Ansprüche 11 bis 13, dadurch gekennzeichnet, daß zumindest ein Teil des aus dem Reaktor 1 abgezogenen Gases erneut dem Reaktor 1 zugeführt wird.

15. Vorrichtung zur Durchführung des Verfahrens nach einem der Ansprüche 1 bis 14 mit mindestens einem Reaktor (1) mit einer Zuleitung (2) für die zu entfernenden unerwünschten Abgasbestandteile, dadurch gekennzeichnet, daß der Reaktor (1) mit einer Flüssigkeit gefüllt ist und daß in der Flüssigkeit einzelne makroporöse Stoffteilchen, auf denen Mikroorganismen angesiedelt sind, in stückiger und/oder granulierter Form frei beweglich schweben.

16. Vorrichtung nach Anspruch 15, dadurch gekennzeichnet, daß dem Reaktor (1) ein eine Zuleitung (9) für zu reinigendes Abgas und eine Ableitung (14) für gereinigtes Abgas aufweisender Sprühturm (10) vorgeschaltet ist, der über die Zuleitung (2) für die zu entfernenden unerwünschten Abgasbestandteile mit dem Reaktor (1) verbunden ist, und daß der Sprühturm (10) Sprühdüsen (12) aufweist, die mit einer Zuleitung (11) für die Flüssigkeit verbunden sind.

## Claims

1. A process for the purification of exhaust gas, wherein undesired exhaust gas constituents are converted into harmless products by micro-organisms, characterised in that the undesired exhaust gas constituents are fed to at least one reactor (1) filled with liquid, wherein individual carrier particles (4) in the form of lumps and/or granules on which micro-organisms have colonized are suspended in the liquid so as to be freely mobile.

2. A process as claimed in Claim 1, characterised in that turbulence is produced in the liquid.

3. A process as claimed in Claim 1 or 2, characterised in that particles of matter with a size of 5 to 50 mm, with open macro-pores of 0.1 to 3 mm and with a specific weight in the dry state of 10 to 200 $kg/m^3$ are used as carrier particles (4).

4. A process as claimed in one of Claims 1 to 3, characterised in that the exhaust gas contains ammonia and/or phenol-formaldehyde compounds as undesired exhaust gas constituents.

5. A process as claimed in one of Claims 1 to 4, characterised in that an aqueous solution containing nutrient salts for the micro-organisms is used as liquid.

6. A process as claimed in one of Claims 1 to 5, characterised in that the reactor (1) is operated under aerobic conditions.

7. A process as claimed in one of Claims 1 to 6, characterised in that the undesired exhaust gas constituents are firstly scrubbed out of the exhaust gas by spraying with liquid in a scrubbing stage (10) arranged upstream of the reactor (1) and are fed to the reactor (1) together with the liquid.

8. A process as claimed in Claim 6 or 7, characterised in that a denitrification stage (15) operated under anoxic conditions is arranged upstream of the reactor (1) operated under aerobic conditions, where activated sludge, containing free micro-organisms, from the reactor (1) is recycled into said denitrification stage (15).

9. A process as claimed in Claim 7 or 8, characterised in that activated sludge, containing free micro-organisms, from the reactor (1) is sprayed together with the liquid in the scrubbing stage (10).

10. A process as claimed in one of Claims 6 to 9, characterised in that air or a gas enriched with oxygen is introduced into the reactor (1) in such manner that turbulence is produced in the liquid.

11. A process as claimed in one of Claims 1 to 5, characterised in that the whole of the exhaust gas is directly input into the liquid contained in the reactor (1).

12. A process as claimed in Claim 11, characterised in that the exhaust gas is introduced into the liquid in such manner that turbulence is produced in the liquid.

13. A process as claimed in Claim 11 or 12, characterised in that a gas containing oxygen is admixed with the exhaust gas.

14. A process as claimed in one of Claims 11 to 13, characterised in that at least a part of the gas discharged from the reactor (1) is returned to the reactor (1).

15. A device for the implementation of the process claimed in one of Claims 1 to 14 comprising at least one reactor (1) with a supply line (2) for the undesired exhaust gas constituents which are to be eliminated, characterised in that the reactor (1) is filled with a liquid and that individual macro-porous particles of matter, on which micro-organisms have colonized, in the form of lumps and/or granules are suspended in the liquid so as to be freely mobile.

16. A device as claimed in Claim 15, characterised in that a spraying column (10) comprising a supply line (9) for exhaust gas which is to be purified and a discharge line (14) for purified exhaust gas is arranged upstream of the reactor (1), which spraying column is connected to the reactor (1) via the supply line (2) for the undesired exhaust gas constituents which are to be eliminated, and that the spraying column (10) is provided with spraying nozzles (12) which are connected to a supply line (11) for the liquid.

**Revendications**

1. Procédé d'épuration des gaz d'échappement, dans lequel la partie indésirable des gaz d'échappement est transformée par des micro-organismes en produits inoffensifs, caractérisé en ce que la partie indésirable des gaz d'échappement est conduite à au moins un réacteur à remplissage liquide (1) dans lequel des particules support individuelles (4) sous forme de morceaux et/ou de granulés, sur lesquelles sont ensemencés des micro-organismes, flottent librement dans le liquide.

2. Procédé selon la revendication 1, caractérisé en ce que dans le liquide des turbulences sont produites.

3. Procédé selon la revendication 1 ou 2, caractérisé en ce qu'on utilise comme particules support des particules de matière dont la taille est comprise entre 5 et 50 mm et qui présentent des macropores dont l'ouverture est comprise entre 0,1 et 3 mm et un poids spécifique à l'état sec de 10 à 200 kg/m$^3$.

4. Procédé selon l'une des revendications 1 à 3, caractérisé en ce que le gaz d'échappement contient comme partie indésirable de gaz d'échappement, de l'ammoniac et/ou des liaisons phénol-formaldéhyde.

5. Procédé selon l'une des revendications 1 à 4, caractérisé en ce qu'on utilise comme liquide une solution aqueuse contenant des sels nutritifs pour les micro-organismes.

6. Procédé selon l'une des revendications 1 à 5, caractérisé en ce que le réacteur (1) fonctionne dans des conditions aérobies.

7. Procédé selon l'une des revendications 1 à 6, caractérisé en ce que la partie indésirable des gaz d'échappement est tout d'abord lavée par aspersion avec le liquide dans un laveur placé en amont du réacteur (1) et conduit ensuite avec le liquide dans le réacteur (1).

8. Procédé selon la revendication 6 ou 7, caractérisé en ce qu'est branché, en amont du réacteur (1) fonctionnant dans des conditions aérobies, un étage de dentrification fonctionnant dans des conditions anoxiques dans lequel sont ramenées des boues activées contenant des micro-organismes provenant du réacteur (1).

9. Procédé selon la revendication 7 ou 8, caractérisé en ce que la boue activée contenant des micro-organismes libres provenant du réacteur (1) est pulvérisée avec le liquide dans le laveur.

10. Procédé selon l'une des revendications 6 à 9, caractérisé en ce que l'on envoie, dans le réacteur (1) de l'air ou un gaz enrichi en oxygène, de manière à provoquer les turbulences dans le liquide.

11. Procédé selon l'une des revendications 1 à 5, caractérisé en ce que la totalité des gaz d'échappement est directement injectée dans le liquide se trouvant dans le réacteur (1).

12. Procédé selon la revendication 11, caractérisé en ce que l'on envoie le gaz d'échappement dans le liquide de façon à ce que les turbulences y sont engendrées.

**13.** Procédé selon la revendication 11 ou 12, caractérisé en ce qu'on ajoute aux gaz d'échappement un gaz contenant de l'oxygène.

**14.** Procédé selon l'une des revendications 11 à 13, caractérisé en ce qu'au moins une partie des gaz extraits du réacteur (1) sont de nouveau injectés dans le réacteur (1).

**15.** Dispositif pour la mise en oeuvre du procédé selon l'une des revendications 1 à 14, ayant au moins un réacteur (1) ayant une conduite d'alimentation (2), pour la partie de gaz d'échappement indésirable qui doit être éliminée, caractérisé en ce que le réacteur est rempli par un liquide et en ce que dans le liquide des particules de matières macroporeuses individuelles, sur lesquelles sont ensemencés des micro-organismes, flottent librement sous forme de blocs et/ou de granulés.

**16.** Dispositif selon la revendication 15, caractérisé en ce que sont montés en amont du réacteur (1) une conduite d'alimentation (9) pour les gaz d'échappement à épurer et une dérivation (14) pour les gaz d'échappement purifiés provenant de la tour de pulvérisation (10), qui sont reliées au réacteur (1) via la conduite d'alimentation (2) pour la partie indésirable de gaz d'échappement qui doit être éliminée, et en ce que la tour de pulvérisation (10) comporte des buses de pulvérisation, qui sont reliées à une conduite (11) d'alimentation en liquide.

Fig.1

Fig.2

Fig.3

Fig.4